(19) Europäisches Patentamt
European Patent Office
Office européen des brevets

(11) Publication number : 0 343 904 B1

# (12) EUROPEAN PATENT SPECIFICATION

(45) Date of publication of patent specification : 28.04.93 Bulletin 93/17

(51) Int. Cl.$^5$ : A61K 31/557

(21) Application number : 89305163.1

(22) Date of filing : 22.05.89

## (54) Hypersphyxia causing composition.

(30) Priority : 23.05.88 JP 125303/88
20.07.88 JP 182281/88

(43) Date of publication of application : 29.11.89 Bulletin 89/48

(45) Publication of the grant of the patent : 28.04.93 Bulletin 93/17

(84) Designated Contracting States : AT BE CH DE ES FR GB GR IT LI LU NL SE

(56) References cited :
EP-A- 0 153 858
CHEMICAL ABSTRACTS, vol. 74, no. 23, 07 June 1971, Columbus, OH (US); J.NAKANO, p. 78, no. 120819y*
CHEMICAL ABSTRACTS, vol. 76, no. 21, 22 May 1972, Columbus, OH (US); J.NAKANO, p. 75, no. 122009z*
PROSTAGLANDINS, vol. 4, no. 6, December 1973; A.A.MATHE et al., pp. 877-890*

(73) Proprietor : Kabushiki Kaisha Ueno Seiyaku Oyo Kenkyujo
4-8, 2-chome, Koraibashi Chuo-ku
Osaka-shi Osaka-fu (JP)

(72) Inventor : Ueno, Ryuzo
10-27 Nango-cho
Nishinomiya-shi Hyogo-Ken (JP)
Inventor : Ueno, Ryuji
7-29 Misaku-cho
Nishinomiya-shi Hyogo-Ken (JP)
Inventor : Oda, Tomio
B-203, 1-29 Suzukakedi
Sanda-shi Hyogo-Ken (JP)

(74) Representative : Atkinson, Peter Birch et al
MARKS & CLERK Suite 301 Sunlight House
Quay Street
Manchester M3 3JY (GB)



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a hypersphyxia-causing composition containing 15-keto-prostaglandin E esters and their derivatives.

In the present specification the term "hypersphyxia-causing compositions" means a composition having an activity of progressing breath, increasing a heart rate, increasing blood pressure, or increasing blood flow rate.

15-keto-prostaglandin E (noted as 15-keto-PGE hereinafter) and 13,14-dihydro-15-keto-prostaglandin E (noted as 13,14-dihydro-15-keto-PGE hereinafter) are known as a substance naturally produced by an enzyme in the metabolism of prostaglandin E (noted as PGE hereinafter) in a living body.

EP-A-0 153 858 (Upjohn) discloses the use of certain 15-keto-PGE esters for the treatment or prevention of acute respiratory distress syndrome or multiple vital organ damage in a human. The treatment of shock, trauma, sepsis or any combination thereof is also disclosed. It has never been recognised that 15-keto-PGEs have hypersphyxia activity.

Fig. 1 is a graph showing an effect of 13,14-dihydro-6,15-diketo-19-methyl-$PGE_1$ ethyl ester on blood flow rate (BF), blood pressure (BP) and heart rate (HR), and

Figs. 2 and 3 are graphs showing an effect of 13,14-dihydro-6,15-diketo-19-methyl-$PGE_1$ ethyl ester on recovery of low blood pressure when dehematized, and on recovery of heart rate respectively.

It has been found that 15-keto-PGEs esters have an activity of increasing blood pressure, a heart rate, and a blood flow rate in, for instance, haemorrhage shock, and hyperventilation.

According to a first aspect of the present invention there is provided the use of a composition comprising 15-keto-PGE ester and a pharmaceutically acceptable carrier for the manufacture of a medicament effective for increasing blood pressure.

According to a second aspect of the present invention there is provided the use of a composition comprising a 15-keto-PGE ester and a pharmaceutically acceptable carrier for the manufacture of a medicament effective for increasing heart rate.

According to a third aspect of the present invention there is provided the use of a composition comprising a 15-keto-PGE ester and a pharmaceutically acceptable carrier for the manufacture of a medicament effective for increasing blood flow rate.

According to a fourth aspect of the present invention there is provided the use of a composition comprising a 15-keto-PGE ester and a pharmaceutically acceptable carrier for the manufacture of a medicament effective for progressing breath.

Especially useful 15-keto-PGE esters for the hypersphyxia are compounds having one or two alkyl group(s), particularly methyl group(s) on 3-, 16- and/or 19-position or one or two halogen atom(s), particularly fluorine atom(s) on 16-position.

This invention provides a hypersphyxia-causing composition which comprises 15-keto-PGE esters as an active ingredient.

In this invention, 15-keto-PGE esters include esters of 15-keto-PGEs of which the carbon atom of 15-position constitutes a carbonyl group, and 13,14-dihydro-15-keto-PGEs of which the bond between carbon atoms of 13- and 14-position is saturated and the carbon atom of 15-position constitutes a carbonyl group. Therefore, this invention includes every prostaglandin E as long as it has in 15-keto or 13,14-dihydro-15-keto form in the prostaglandin skeleton structure and is not limited by other additional skeleton structure or substituents.

In the present specification 15-keto-PGEs are expressed according to the following nomenclature. The 15-keto-PGEs have a following basic structure:

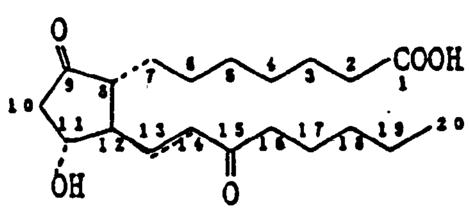

( α-chain)

( ω-chain)

and the position number of carbon atom constituting α-chain, ω-chain and five membered ring in the prostaglandin skeleton structure is used as it is in the drawing. That is, the position number of the carbon atom in the skeleton structure is started from the carbon atom constituting carboxylic acid of the terminal position of α-chain through the five membered ring to ω-chain, i.e. 1 to 7 are attached to the carbon atoms in the α-chain in this order, 8 - 12 are attached to the carbon atoms in the five membered ring, and 13 - 20 are attached to

the carbon atoms in the ω-chain. In the compound whose carbon number in α-chain is less than 7 the position number is simply eliminated from 2 to 7 in this order without any change of the position number of the other carbons. In other word 15-keto-PGEs having 6 carbon atoms in the α-chain have no position of 2, i.e. 15-keto-PGEs of such compound are not renamed as 14-keto-PGEs. In case that carbon atoms increase in the α-chain the carbon chain increased is nominated as a substituent on the carbon of position number 2 without any change of the position number of the other carbons. Therefore, 15-keto-PGEs having 8 carbon atoms in the α-chain are nominated as 15-keto-2-decarboxy-2-acetic acid-PGEs. In case that the number of the carbon in the ω-chain decreases the position number is nominated as reducing it from the carbon of position number 20 one by one. In case the number of carbon atoms in the ω-chain increases, the increased carbon chain is nominated as a substituent on the carbon of position number 20. That is, 15-keto-PGEs having 10 carbon atoms in the ω-chain is nominated as 15-keto-20-ethyl-PGEs.

The above formula expresses a specific configuration which is most typical one, and in this specification compounds having such a configuration are expressed unless otherwise described.

PGEs have a hydroxy group on the 11-position in general, but in the present specification term "PGEs" includes prostaglandins having another group instead of said hydroxyl group of normal PGE. Such PGEs are called as 11-dehydroxy-11-substituent-PGEs, for instance, 11-dehydroxy-11-methyl-PGEs in case of the substituent being a methyl group.

PGEs are classified to $PGE_1$ and $PGE_2$ according to the bonds between 5- and 6-positions.

$PGE_1$ and its derivatives (referred to as $PGE_1$s hereinafter) are nominated to a group of compounds in which the bond between 5- and 6-positions is a single bond. $PGE_2$ and its derivatives (referred to as $PGE_2$s hereinafter) are called to a group of compounds in which the bond between 5- and 6-positions is a cis-double bond. PGEs having a structure of

$$-CH_2-C(O)-CH_2-$$
7 6 5

is nominated as 6-keto-$PGE_1$s, and PGEs having a structure of

$$-CH_2-C{\equiv}C-$$
7 6 5

is called as 5,6-dehydro-$PGE_2$s.

The hypersphyxia-causing activity is remarkably expressed in 15-keto-PGEs esters represented by the following formula:

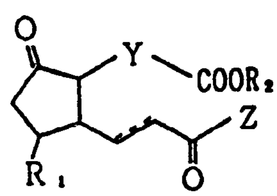

wherein $R_1$ is a hydroxyl group, a hydroxyalkyl group, or an alkyl group; Y is a saturated or unsaturated hydrocarbon moiety having 2 - 6 carbon atoms wherein a portion of carbon atoms constituting the hydrocarbon moiety may be carbonyl carbon or a portion of hydrogen atoms attached to the hydrocarbon moiety may be substituted with other atoms or groups; Z is a saturated or an unsaturated hydrocarbon moiety which may constitute a straight chain or a ring, wherein a portion of hydrogen atoms in the hydrocarbon moiety may be substituted with other atoms or groups; $COOR_2$ is a physiologically acceptable ester group.

Y represents a saturated or an unsaturated hydrocarbon moiety having 2 - 6 carbon atoms includes an aliphatic hydrocarbon such as an alkylene group, an alkenylene group and, an alkynylene group. Y may preferably be a hydrocarbon chain having 6 carbon atoms.

Examples of PGEs of which Y is an unsaturated hydrocarbon moiety are $PGE_2$s, 5,6-dehydro-$PGE_2$s, PGEs of which the bond between 2- and 3-positions is a double-bond, and the like.

A portion of carbon atoms constituting hydrocarbon moiety represented by Y may be a carbonyl group, whose typical examples are 6-keto-$PGE_1$s in which the carbon atom of 6-position is a carbonyl group.

The hydrocarbon moiety represented by Y may be substituted with one or more other atom(s) or group(s), for example, halogen atoms such as a fluorine atom, a chlorine atom, typically a fluorine atom; an alkyl group

such as methyl, ethyl; a hydroxyl group. Typical examples of such substituents are 15-keto-PGEs having one or more alkyl group(s) on the carbon atom of 3-position.

Z represents a saturated or an unsaturated hydrocarbon moiety having 1 - 10 carbon atoms. The hydrocarbon moiety may be an aliphatic hydrocarbon or a cyclic hydrocarbon itself or in part. The hydrocarbon moiety represented by Z may be substituted with one or more other atom(s) or group(s).

The number of the carbon atoms of Z is preferably 3 - 7 in straight chain. PGEs in which carbon numbers of Z are 5 are typical PGs. Therefore, the PGEs in which carbon numbers of the hydrocarbon moiety represented by Z are 6 or more than 6 are nominated as PGEs having a substituent on the carbon atom of 20-position. That is, PGEs in which the number of carbon atoms of Z is 6 are nominated as 20-methyl-PGEs.

Though the hydrocarbon moiety represented by Z may have one or more unsaturated bond(s) at any position, a saturated hydrocarbon is more preferable. Examples of the hydrocarbon moiety having a cycloalkyl group are a cyclopentyl or a cyclohexyl containing a carbon atom itself of 16- or 17-position as a ring constituting member.

The hydrocarbon moiety represented by Z may be substituted with one or more other atom(s) or group(s), for example, a halogen atom such as a fluorine atom or a chlorine atom; an alkyl group such as methyl, ethyl, isopropyl, isopropenyl; an alkoxy group such as methoxy, ethoxy; a hydroxyl group; a phenyl group; a phenoxy group. The substituent(s) may be preferably located at the 16-, 17-, 19- and/or 20-position, but not restricted. Examples of preferable compounds include those having one or two, different or identical atom(s) and/or groups, for example, a halogen atom such as a fluorine atom; an alkyl group such as a methyl, an ethyl group; an aromatic group which may have substituents such as a methyl, ethyl, phenyl, benzyl, phenoxy, hydroxyl or other group on 16-position. Other examples of preferable compounds include those having a cycloalkyl group such as a cyclopentyl or cyclohexyl group which contains the carbon atom of 16-position as a constituent of the cyclic ring; an alkyl group such as methyl, ethyl and the like on 17- or 19-position; an alkyl group such as methyl, ethyl, isopropyl, isopropenyl and the like; an alkoxy group such as methoxy, ethoxy, propoxy and the like on 20-position.

A generic name of PGEs is used to compounds having a prostanoic acid structure in which the carbon atom of 11-position has a hydroxyl group, and the carbon atom of 9-position forms a carbonyl group. In the present specification prostaglandins derivatives in which the hydroxyl group on 11-position is substituted with a hydroxyalkyl group or an alkyl group is also called as PGEs. Therefore, the 15-keto-PGEs of the present invention include compounds in which $R_1$ of the general formula (I) represents a hydroxyalkyl group or an alkyl group, for example, as a hydroxyalkyl group hydroxymethyl, 1-hydroxyethyl, 2-hydroxyethyl and 1-methyl-1-hydroxyethyl groups; as an alkyl group methyl and ethyl groups are preferably exemplified.

The steric configuration of $R_1$ with respect to the carbon of 11-position may be $\alpha$ or $\beta$ or mixture thereof.

Furthermore, examples of the PGEs included in the present invention are 13,14-dihydro compounds which have saturated bond between the 13- and 14-positions, and rather more preferable effects can be achieved from such 13,14-dihydro compounds.

The 15-keto-PGEs of the present invention is esterified on the terminal carboxyl group.

Useful ester ($COOR_2$) of 15-keto-PGEs may include a saturated or an unsaturated lower alkyl ester which may have a branched chain such as methyl, ethyl, propyl, n-butyl, isopropyl, t-butyl, 2-ethylhexyl, allyl and the like; an aliphatic cyclic ester such as cyclopropyl, cyclopentyl, cyclohexyl and the like; an aromatic ester such as benzyl, phenyl and the like, which may have substituents; a hydroxyalkyl or an alkoxyalkyl ester such as hydroxyethyl, hydroxyisopropyl, hydroxypropyl, polyhydroxyethyl, polyhydroxyisopropyl, methoxyethyl, ethoxyethyl, methoxyisopropyl and the like; a trialkylsilyl ester such as trimethylsilyl, triethylsilyl and the like; a heterocyclic ester such as tetrahydropyranyl and the like. Preferable esters for the present invention are a lower alkyl ester which may have a branched chain, for instance, methyl, ethyl, propyl, n-butyl, isopropyl, t-butyl; a benzyl ester; a hydroxyalkyl ester such as hydroxyethyl, hydroxy isopropyl. In the alkyl ester remarkable results of hypersphyxia can be usually obtained.

15-keto-PGEs of the present invention may include various kinds of isomers such as tautomeric isomers, optical isomers and, geometric isomers. As an example of such isomers there is exemplified a keto-hemiacetal tautomeric isomer between the hydroxyl group on 11-position and the carbonyl group of 15-position of 15-keto-PGEs. The latter tautomeric isomer is liable to be caused in 15-keto-PGEs having an electron withdrawing group such as fluorine atom on 16-position.

A hemiacetal, a tautomeric isomer between the hydroxyl group on 11-position and the carbonyl group of 15-position, may be sometimes formed, and an equilibrium mixture of the compound of $R_1$ being a hydroxyl group and a hemiacetal may be given. Such an equilibrium mixture or a tautomeric isomer is also included in the 15-keto-PGEs of the present invention.

An equilibrium mixture of the isomers such as racemic mixture, tautomers of a hydroxyl compound and a hemiacetal also exhibits similar effects to a single compound.

Examples of especially useful compounds for an accelerator of heart rate in hypersphyxia include compounds having one or two alkyl group(s), especially methyl group(s); halogen atom(s), especially fluorine atom(s) at 3-position, 16-position and/or 19-position; compounds in which $R_1$ is methyl; compounds in which the carbon number of the main chain of ω-chain is 8 to 10; the bond between 2- and 3-positions is double-bond.

The examples of 15-keto-PGEs especially effective to increase of heart rate are 13,14-dihydro-15-keto-$\Delta^2$-$PGE_1$ alkyl ester, 13,14-dihydro-15-keto-20-ethyl-$PGE_1$ alkyl ester, 13,14-dihydro-6,15-diketo-16,16-dimethyl-$PGE_1$ alkyl ester, 13,14-dihydro-6,15-diketo-16R,S-fluoro-$PGE_1$ alkyl ester, 13,14-dihydro-6,15-diketo-11-dehydroxy-11R-methyl-$PGE_1$ alkyl eater, 13,14-dihydro-6,15-diketo-16R,S-fluoro-11-dehydroxy-11R-methyl-$PGE_1$ alkyl ester, 13,14-dihydro-15-keto-$PGE_2$ alkyl ester, 13,14-dihydro-15-keto-3R,S-methyl-$PGE_2$ alkyl ester, 13,14-dihydro-15-keto-16R,S-methyl-$PGE_2$ alkyl ester, 13,14-dihydro-15-keto-3R,S,16R,S-dimethyl-$PGE_2$ alkyl ester, 13,14-dihydro-15-keto-16R,S-fluoro-$PGE_2$ alkyl ester, 13,14-dihydro-15-keto-16R,S-fluoro-20-methyl-$PGE_2$ alkyl ester, 13,14-dihydro-15-keto-19-methyl-$PGE_2$ alkyl ester, 13,14-dihydro-15-keto-20-methoxy-16,16-dimethyl-$PGE_2$ alkyl ester, 15-keto-16R,S-fluoro-$PGE_2$ alkyl ester.

Examples of 15-keto-PGEs especially useful for hypertension are compounds of $R_1$ being methyl; compounds of which the bond between 2- and 3-positions is a double bond; compounds in which possess one or two halogen atom(s) or alkyl group(s), especially fluorine atom(s) or methyl group(s) is bound on 16- and/or 19-position; compounds in which carbon number of main chain in the ω-chain is 8 - 12.

Concrete examples of these preferable compounds are 13,14-dihydro-15-keto-$\Delta^2$-$PGE_1$ alkyl ester, 13,15-dihydro-6,15-diketo-16,16-dimethyl-$PGE_1$ alkyl ester, 13,14-dihydro-6,15-diketo-16R,S-methyl-$PGE_1$ alkyl ester, 13,14-dihydro-6,15-diketo-16R,S-fluoro-$PGE_1$ alkyl ester, 13,14-dihydro-6 ,15-diketo-11-dehydroxy-11R-methyl-$PGE_1$ alkyl ester, 13,14-dihydro-6,15-diketo-16R,S-fluoro-11-dehydroxy-11R-methyl-$PGE_1$ alkyl ester, 13,14-dihydro-15-keto-$PGE_2$ alkyl ester, 13,14-dihydro-15-keto-16,16-dimethyl-$PGE_2$ alkyl ester, 13,14-dihydro-15-keto-16R,S-fluoro-11-dehydroxy-11R-methyl-$PGE_2$ alkyl ester, 13,14-dihydro-15-keto-16R,S-hydroxy-$PGE_2$ alkyl ester, 13,14-dihydro-15-keto-16R,S-fluoro-$PGE_2$ alkyl ester, 13,14-dihydro-15-keto-16R,S-fluoro-20-methyl-$PGE_2$ alkyl ester, 13,14-dihydro-15-keto-11-dehydroxy-11R-methyl-$PGE_2$ alkyl ester, 13,14-dihydro-15-keto-19-methyl-$PGE_2$ alkyl ester, 13,14-dihydro-15-keto-20-methoxy-16,16-dimentyl-$PGE_2$ alkyl ester, 13,14-dihydro-15-keto-20-ethyl-11-dehydroxy-11R-methyl-$PGE_2$ alkyl ester and 13,14-dihydro-15-keto-20-n-propyl-$PGE_2$ alkyl ester.

Examples of especially useful compounds for hyperventilation include compounds having one or two alkyl group(s), especially methyl; halogen atom(s), especially fluorine atom(s) at 3-position, 16-position and/or 19-position; compounds of which $R_1$ is methyl; and compounds of which the carbon number of the main chain of ω-chain is 8 to 10.

The examples of 15-keto-PGEs especially effective to increase of heart rate are 13,14-dihydro-15-keto-$PGE_1$ alkyl ester, 13,14-dihydro-15-keto-20-ethyl-$PGE_1$ alkyl ester, 13,14-dihydro-6,15-diketo-$PGE_1$ alkyl ester, 13,14-dihydro-6,15-diketo-16R,S-methyl $PGE_1$ alkyl ester, 13,14-dihydro-6,15-diketo-16,16-dimethyl-$PGE_1$ alkyl ester, 13,14-dihydro-6,15-diketo-16R,S-fluoro-$PGE_1$ alkyl ester, 13,14-dihydro-15-keto-3R,S-methyl-$PGE_2$ alkyl ester, 13,14-dihydro-15-keto-16R,S-methyl-$PGE_2$ alkyl ester, 13,14-dihydro-15-keto-3R,S,16R,S-dimethyl-$PGE_2$ alkyl ester, 13,14-dihydro-15-keto-16,16-dimethyl-$PGE_2$ methyl ester, 13,14-dihydro-15-keto-20-ethyl-$PGE_2$ methyl ester, 13,14-dihydro-15-keto-16R,S-fluoro-$PGE_2$ alkyl ester, and 13,14-dihydro-15-keto-19-methyl-$PGE_2$ alkyl ester.

Examples of 15-keto-PGEs preferred in the aspect of heart rate, blood pressure increasing abilities and breath progressing activity are compounds having one or two alkyl group(s), especially methyl group(s) on 3-, 16- and/or 19-position or one or two halogen atom(s), especially fluorine atom(s) on 16-position.

Concrete examples of these preferable compounds are 13,14-dihydro-6,15-diketo-16-R,S-methyl-$PGE_1$ alkyl ester, 13,14-dihydro-6,15-diketo-16,16-dimethyl-$PGE_1$ alkyl ester, 13,14-dihydro-6,15-diketo-16R,S-fluoro-$PGE_1$ alkyl ester, 13,14-dihydro-15-keto-3R,S-methyl-$PGE_2$ alkyl ester, 13,14-dihydro-15-keto-16R,S-methyl-$PGE_2$ alkyl ester, 13,14-dihydro-15-keto-3R,S,16R,S-dimethyl-$PGE_2$ alkyl ester, 13,14-dihydro-15-keto-16,16-dimethyl-$PGE_2$ alkyl ester, 13,14-dihydro-15-keto-16R,S-fluoro-$PGE_2$ alkyl ester, and 13,14-dihydro-15-keto-19-methyl-$PGE_2$ alkyl ester.

Most preferable compounds are those having one or two lower alkyl group(s) such as methyl or ethyl group(s) or hydroxyl group(s) on 16-position because of their excellent blood pressure increasing ability and cardiotonic action.

In the present specification PGEs are named based on a prostanoic acid skeleton, but it can be named according to IUPAC nomenclature, according to which, for instance, $PGE_1$, is nominated as 7-{(1R,2R,3R)-3-hydroxy-2-[(E)-(3S)-3-hydroxy-1-octenyl]-5-oxo-cyclopentyl}-heptanoic acid; $PGE_2$ is nominated as (Z)-7-{(1R,2R,3R)-3-hydroxy-2-[(E)-(3S)-3-hydroxy-1-octenyl]-5-oxo-cyclopentyl}-hept-5-enoic acid; 13,14-dihydro-15-keto-16R,S-fluoro-$PGE_2$ is nominated as (Z)-7-{(1R,2R,3R)-3-hydroxy-2-[(4R,S)-4-fluoro-3-oxo-1-octyl]-5-oxo-cyclopentyl}-hept-5-enoic acid; 13,14-dihydro-15-keto-20-ethyl-11-dehydroxy-11R-methyl $PGE_2$ methyl ester is nominated as Methl (z)-7-{(1R,2S,3R)-3-methyl-2-[3-oxo-1-decyl]-5-oxo-cyclopentyl}-hept-5-enoate; and 13,14-dihydro-6,15-diketo-19-methyl-$PGE_1$ ethyl ester is nominated as ethyl 7-{(1R,2R,3R)-3-hydroxy-2-(7-methyl-3-oxo-1-octyl)-5-oxo-cyclopentyl}-6-oxo-heptanoate.

The 15-keto-PGEs used in this invention may be prepared, for example, by the method noted in Japanese Patent Application No. 18326/1988.

A practical preparation of the 13,14-dihydro-15-keto PGEs involves the following steps; as shown in the synthetic charts (I) to (III), reaction of the aldehyde (2) prepared by the Collins oxidation of commercially available (-)-Corey lactone (1) with dimethyl (2-oxoheptyl)phosphate anion to give α,β-unsaturated ketone (3), reaction of the α,β- unsaturated ketone (3) to the corresponding saturated ketone (4), protection of the carbonyl group of the ketone (4) with a diol to the corresponding ketal (5), and deprotection of the p-phenylbenzoyl group to give the corresponding alcohol (6) followed by protection of the newly generated hydroxy group with dihydropyrane to give the corresponding tetrahydropyranyl ether (7). According to the above process, a precursor of PGEs of which ω-chain is a 13,14-dihydro-15-keto-alkyl group is prepared.

A precursor of PGEs of which ω-chain is 15-keto-alkyl group can be obtained by proceeding the reaction without reduction of α,β-unsaturated ketons (3).

Using the above tetrahydropyranyl ether (7), 6-keto-$PGE_1$s (15) of which a group constituted with carbon atoms of 5-, 6- and 7-position is

$$\underset{7}{-CH_2}-\underset{6}{C(O)}-\underset{5}{CH_2}-,$$

may be prepared in the following steps; reduction of the tetrahydropyranyl ether (7) with, for example, diisobutyl aluminum hydride to give the corresponding lactol (8), reaction of the lactol (8), with the ylide generated from (4-carboxybutyl)triphenyl phosphonium bromide followed by esterification (10), cyclization between the 5,6-double bond and the hydroxyl group at 9-position with NBS or iodine to give the halogenated compound (11), dehydrohalogenation of the compound (11) with, for example, DBU to give the 6-keto compound (13) followed by Jones oxidation and removal of the protecting groups.

Furthermore, $PGE_2$s (19) of which a group constituted with carbon atoms of 5-,6- and 7-position is

$$\underset{7}{-CH_2}-\underset{6}{CH}=\underset{5}{CH}-$$

may be prepared in the following steps; as shown in the synthetic chart II, reduction of the above the tetrahydropyranyl ether (7) to give the lactol (8), reaction of the resultant lactol (8) with the ylide generated from (4-carboxybutyl)triphenyl phosphonium bromide to give the carboxylic acid (16) followed by esterification to give ester (17), Jones oxidation of the esters (17) to give the compound (18), and removal of the protecting groups.

Using the above the tetrahydropyranyl ether (7) as starting material, the compound having

$$\underset{7}{-CH_2}-\underset{6}{CH_2}-\underset{5}{CH_2}-$$

may be prepared by using the same process for preparing $PGE_2$s having the group of

$$\underset{7}{-CH_2}-\underset{6}{CH}=\underset{5}{CH}-$$

and applying the resultant compound (18) to catalytic reduction for reducing the double bond between the 5- and 6-position followed by removal of the protection groups.

Synthesis of 5,6-dehydro-$PGE_2$s having

$$\underset{7}{-CH_2}-\underset{6}{C}\equiv\underset{5}{C}-$$

may be carried out by alkylation of the resulting a copper enolate generated after 1,4-addition of a monoalkylcopper complex or a dialkylcopper complex of the following formula:

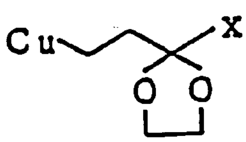 , 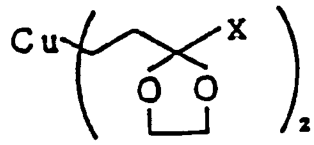

to 4R-t-butyldimethylsilyloxy-2-cyclopenten-1-one with 6-alkoxycarbonyl-1-iodo-2-hexyne or the derivatives.

The 11-β type PGEs can be prepared according to the synthetic chart III.

PGEs having a methyl group on 11-position instead of a hydroxyl group can be obtained by reacting PGAs with a dimethyl copper complex, which are prepared by Jones oxidation of a hydroxyl group on the 9-position of 11-tosylates. PGEs having a hydroxymethyl group on 11-position instead of a hydroxyl group can be prepared by photoaddition of methanol to the PGAs using benzophenone as a photosensitizer.

The 15-keto-PGEs of this invention may be used as a medicine for animals and human beings and usually applied systemically or locally by the method of oral administration, oral administration by spraying, intravenous injection (including instillation), subcutaneous injection, suppository and the like. Dose is determined depending on the animal to be treated, the human patient, age, body weight, symptom, therapeutic effect, administration route, and treating time, but is preferably 0.001 - 500 mg/kg.

As solid composition of this invention for oral administration, tablets, capsules, powders, granules and the like are included. The solid composition containing one or more active substances is mixed with at least an inactive diluent such as lactose, mannitol, glucose, hydroxypropyl cellulose, fine crystalline cellulose, starch, polyvinyl pyrolidone, magnesium aluminate metasilicate. The composition may contain except for the inactive diluent other additives such as lubricants (e.g. magnesium stearate), a disintegrator (e.g. cellulose calcium gluconate), a stabilizer (e.g. α-, β- or γ-cyclodextrin, etherated dextrin (e.g. dimethyl-α-, dimethyl-β-, trimethyl-β- or hydroxypropyl-β-cyclodextrin), branched cyclodextrin (e.g. glucosyl- or maltosyl-cyclodextrin), formyl cyclodextrin, sulfur-containing cyclodextrin or misoprotol). Such cyclodextrins may form an inclusion compound with 15-keto-PGEs in some cases to increase the stability of the compounds. The stability may be often increased by forming lyposome with phospholipid. Tablets and pills may be coated with an enteric or gastroenteric film such as white sugar, gelatin, hydroxypropylcellulose, hydroxypropylmethylcellulose phthalate and the like, if necessary, and furthermore they may be covered with two or more layers. Additionally, the composition may be in the form of capsules made of substance easily absorbed such as gelatin.

Liquid compositions for oral administration include pharmaceutically acceptable emulsion, solutions, suspension, syrups, elixirs and the like and contain a generally used inactive diluent such as purified water or ethyl alcohol. The composition may contain additives such as wetting agents and suspending agents as well as sweeteners, flavors, aromatics and preservatives.

The compositions for oral administration may contain one or more active substance.

The injection of this invention for non-oral administration includes sterile aqueous or nonaqueous solutions, suspensions, and emulsions. Diluents for the aqueous solution or suspension contain, for example, distilled water for injection, physiological saline and Ringer's solution.

Diluents for the nonaqueous solution and suspension contain, for example, propylene glycol, polyethylene glycol, vegetable oils such as olive oil, alcohols such as ethanol and polysorbates. The composition may contain other additives such as preservatives, wetting agents, emulsifying agents, dispersing agents and the like. These are sterilized by filtration through, e.g. a bacteria-preventing filter, compounding with a sterilizer, gas sterilization or radiation sterilization. These can be prepared by producing a sterile solid composition and dissolving it into sterilized water or a sterilized solvent for injection before use.

As a composition for intrarectal administration there may be included spherical or spindle shape medicines containing one or more active materials and one or more solid carriers such as cacaos or Carbowax®, and a suppository such as gelatin capsules.

The present invention is illustrated by Examples in detail to clarify the effectiveness of the present invention. The term "effective components" used in the Examples means optional PGEs of the present invention.

Example 1 (blood pressure, heart rate increasing action by intravenous injection)

Wister male rats (8 weeks old) were used as test animals.

The rats were applied to anesthesia by intraperitoneal administration with 1.25 g/kg of urethane. The blood pressure was determined by inserting a polyethylene tube into a femoral artery, which was connected with a pressure transducer. The heart rate was determined from R wave of electrocardiograms (ECG) induced by standard limb lead II with a tachometer. Each test compound was dissolved in ethanol and intravenously administered to rats in 1 mg/kg diluted with Ringer's solution just before use. The maximum concentration of ethanol was 2 percent. An ethanol-containing Ringer without any test compound was used as a control to confirm the influence of the ethanol in each experiment. Each changing rate (%) in the blood pressure and heart rate before and after administration was determined, and the average of 3 - 4 experiments was shown in Table 1.

Table 1

| test compound | changing rate of heart rate (%) | | | changing rate of blood pressure (%) | | |
|---|---|---|---|---|---|---|
| | 0.2 | 0.5 | 1 | 0.2 | 0.5 | 1 |
| 1* | 0 | 0 | +6 | 0 | 0 | -6 |
| 2 | +6 | +20 | +20 | 0 | +10 | +1 |
| 3 | | | +43 | | | +32 |
| 4 | +9 | +18 | +33 | +4 | +6 | +6 |
| 5* | 0 | 0 | 0 | 0 | 0 | -4 |
| 6 | +13 | +23 | +9 | +13 | +10 | +3 |
| 7 | +2 | +3 | +9 | +4 | +6 | +5 |
| 8 | +18 | +25 | +28 | +15 | +14 | +8 |
| 9 | 0 | +14 | +26 | 0 | 0 | +2 |
| 10 | +23 | +22 | +37 | +17 | +19 | +22 |
| 11 | +14 | +11 | +18 | +19 | +13 | +23 |
| 12 | +8 | +40 | +38 | +9 | +13 | +17 |
| 13 | +29 | +33 | +20 | +18 | +8 | +11 |
| 14 | +15 | +11 | +22 | +7 | +4 | +12 |
| 15 | +3 | +8 | +19 | +2 | +10 | +12 |
| 16 | | | +25 | | | +26 |
| 17 | | | +58 | | | +46 |
| 18 | +8 | +13 | +17 | +10 | +12 | +18 |
| 19 | | | +51 | | | +45 |
| 20* | 0 | 0 | 0 | 0 | 0 | -15 |
| 21 | +30 | +22 | +35 | +12 | +9 | +18 |
| 22 | 0 | +6 | +11 | 0 | 0 | +5 |
| 23 | 0 | 0 | +4 | 0 | 0 | +5 |
| 24 | 0 | 0 | +5 | 0 | 0 | +5 |

| test compound | changing rate of heart rate(%) | | | changing rate of blood pressure (%) | | |
|---|---|---|---|---|---|---|
| | 0.2 | 0.5 | 1 | 0.2 | 0.5 | 1 |
| 25 | 0 | 0 | +5 | 0 | 0 | +2 |
| 26 | 0 | 0 | +7 | 0 | 0 | +2 |
| 27 | +19 | +20 | +21 | +4 | +8 | +6 |
| 28 | +20 | +30 | +33 | +13 | +14 | +12 |
| 29 | +4 | +7 | +17 | +5 | +6 | +7 |
| 30 | +29 | +20 | +29 | +14 | +15 | +14 |
| 31 | +6 | +7 | +46 | +4 | +4 | +14 |
| 32 | +3 | +16 | +30 | +3 | +7 | +14 |
| 33 | +20 | +28 | +28 | +18 | +29 | +20 |
| 34 | 0 | +14 | +29 | 0 | +3 | +6 |
| 35 | +17 | +18 | +27 | +5 | +12 | +17 |
| 36 | | | +32 | | | +33 |
| 37 | | | +33 | | | +56 |
| 38 | +20 | +33 | +15 | +12 | +18 | +14 |
| 39 | | | +47 | | | +20 |
| 40 | +20 | +16 | +27 | +17 | +19 | +17 |
| 41 | +6 | +12 | +26 | +20 | +20 | +11 |
| 42 | 0 | 0 | +7 | 0 | 0 | +12 |
| 43 | +17 | +27 | +45 | +10 | +10 | +14 |
| 44 | +3 | +17 | +30 | +1 | +9 | +20 |
| 45 | 0 | 0 | +4 | 0 | 0 | +2 |
| 46 | 0 | 0 | +13 | 0 | 0 | +5 |
| 47 | 0 | 0 | +4 | 0 | 0 | +1 |
| 48 | | | +30 | | | +15 |
| 49 | 0 | 0 | +3 | 0 | 0 | +2 |

| test compound | changing rate of heart rate(%) | | | changing rate of blood pressure (%) | | |
|---|---|---|---|---|---|---|
| | 0.2 | 0.5 | 1 | 0.2 | 0.5 | 1 |
| 50 | 0 | 0 | +5 | 0 | 0 | +2 |
| 51 | 0 | 0 | +14 | 0 | 0 | +1 |
| 52 | | | 0 | | | +13 |
| 53 | | | +20 | | | +17 |
| 54 | | | +18 | | | +18 |
| 55 | | | +58 | | | +31 |
| 56* | +8 | +10 | +10 | -37 | -45 | -54 |
| 57* | +13 | +22 | +14 | -35 | -40 | -39 |
| 58* | +10 | +14 | +33 | -27 | -20 | -11 |
| 59* | | 0 | | | 0 | |

* : (comparative example)

Test Compound

1: 13,14-dihydro-15-keto-$PGE_1$
2: 13,14-dihydro-15-keto-$PGE_1$ ethyl ester
3: 13,14-dihydro-15-keto-$\Delta^2$-$PGE_1$ methyl ester
4: 13,14-dihydro-15-keto-20-ethyl-$PGE_1$ methy ester
5: 13,14-dihydro-6,15-diketo-$PGE_1$
6: 13,14-dihydro-6,15-diketo-$PGE_1$ methyl ester
7: 13,14-dihydro-6,15-diketo-$PGE_1$ ethyl ester
8: (±) 13,14-dihydro-6,15-diketo-$PGE_1$ ethyl ester
9: 13,14-dihydro-6,15-diketo-$PGE_1$ n-butyl ester
10: 13,14-dihydro-6,15-diketo-16R,S-methyl-$PGE_1$ methyl ester
11: 13,14-dihydro-6,15-diketo-16R,S-methyl-$PGE_1$ ethyl ester
12: 13,14-dihydro-6,15-diketo-16,16-dimethyl-$PGE_1$ ethyl ester
13: 13,14-dihydro-6,15-diketo-16R,S-fluoro-$PGE_1$ ethyl ester,
14: 13,14-dihydro-6,15-diketo-19-methyl-$PGE_1$ methyl ester
15: 13,14-dihydro-6,15-diketo-19-methyl-$PGE_1$ ethyl ester
16: 13,14-dihydro-6,15-diketo-11-dehydroxy-11R-hydroxymethyl-19-methyl-$PGE_1$ methyl ester
17: 13,14-dihydro-6,15-diketo-11-dehydroxy-11R-methyl-$PGE_1$ methyl ester
18: 13,14-dihydro-6,15-diketo-11-dehydroxy-11R-methyl-$PGE_1$ ethyl ester
19: 13,14-dihydro-6,15-diketo-16R,S-fluoro-11R-dehydroxy-11R-methyl-$PGE_1$ ethyl ester
20: 13,14-dihydro-15-keto-$PGE_2$
21: 13,14-dihydro-15-keto-$PGE_2$ methyl ester
22: 13,14-dihydro-15-keto-$PGE_2$ ethyl ester
23: 13,14-dihydro-15-keto-$PGE_2$ n-propyl ester
24: 13,14-dihydro-15-keto-$PGE_2$ n-butyl ester
25: 13,14-dihydro-15-keto-$PGE_2$ benzyl ester
26: 13,14-dihydro-15-keto-$PGE_2$ hydroxyethyl ester
27: 13,14-dihydro-15-keto-$\Delta^2$-$PGE_2$-methyl ester
28: 13,14-dihydro-15-keto-3R,S-methyl-$PGE_2$ methyl ester

29: 13,14-dihydro-15-keto-3R,S-methyl-$PGE_2$ ethyl ester
30: 13,14-dihydro-15-keto-16R,S-methyl-$PGE_2$ methyl ester
31: 13,14-dihydro-15-keto-16R,S-methyl-$PGE_2$ ethyl ester
32: 13,14-dihydro-15-keto-3R,S-16R,S-dimethyl-$PGE_2$ methyl ester
33: 13,14-dihydro-15-keto-16,16-dimethyl-$PGE_2$ methyl ester
34: 13,14-dihydro-15-keto-16,16-dimethyl-$PGE_2$ ethyl ester
35: 13,14-dihydro-15-keto-16R,S-hydroxy-$PGE_2$ ethyl ester
36: 13,14-dihydro-15-keto-16R,S-fluoro-$PGE_2$ (tautomeric isomer between carbonyl group at carbon atom of 15-position and hydroxyl group on carbon atom of 11-position is confirmed.)
37: 13,14-dihydro-15-keto-16R,S-fluoro-$PGE_2$ methyl ester
38: 13,14-dihydro-15-keto-16R,S-fluoro-$PGE_2$ ethyl ester
39: 13,14-dihydro-15-keto-16R,S-fluoro-20-methyl-$PGE_2$ methyl ester
40: 13,14-dihydro-15-keto-16R,S-fluoro-11-dehydroxy-11R-methyl-$PGE_2$ ethyl ester
41: 13,14-dihydro-15-keto-11-dehydroxy-11R-methyl-$PGE_2$ ethyl ester
42: 13,14-dihydro-15-keto-17S-methyl-$PGE_2$ methyl ester
43: 13,14-dihydro-15-keto-19-methyl-$PGE_2$ methyl ester
44: 13,14-dihydro-15-keto-19-methyl-$PGE_2$ ethyl ester
45: 13,14-dihydro-15-keto-20-methoxy-$PGE_2$ methyl ester
46: 13,14-dihydro-15-keto-20-methoxy-$\Delta^2$-$PGE_2$ methyl ester
47: 13,14-dihydro-15-keto-3R,S-methyl-20-methoxy-$PGE_2$ methyl ester
48: 13,14-dihydro-15-keto-16,16-dimethyl-20-methoxy-$PGE_2$ methyl ester
49: 13,14-dihydro-15-keto-20-isopropylidene-$PGE_2$
50: 13,14-dihydro-15-keto-20-isopropylidene-$PGE_2$ methyl ester
51: 13,14-dihydro-15-keto-20-ethyl-$PGE_2$ methyl ester
52: 13,14-dihydro-15-keto-20-ethyl-$PGE_2$ ethyl ester
53: 13,14-dihydro-15-keto-20-ethyl-11-dehydroxy-11R-methyl-$PGE_2$ methyl ester
54: 13,14-dihydro-15-keto-20-n-propyl-$PGE_2$ methyl ester
55: 15-keto-16R,S-fluoro-$PGE_2$ methyl ester
56: $PGE_1$
57: $PGE_2$
58: $PGE_2$ methyl ester
59: control

The test compound (esters of 15-keto-PGEs) exhibits a blood pressure increasing effect instead of showing a blood pressure decreasing effect which are observed in PGs such as $PGE_2$, $PGE_2$ methyl esters; and 15-keto-PGEs having a free carboxylic acid residue such as 13,14-dihydro-15-keto-$PGE_2$. The esters of 15-keto-PGEs also exhibits positive chronotropic effect (cardiotonic effect).

Example 2

Changing rates of blood pressure and heart rate were determined in the same manner as described in Example 1 except that the test compounds 60, 61 and 62 were used in different concentrations instead of the test compounds 1 - 55. The results were shown in Table 2.

Table 2

| test compound | dose mg/kg | changing rate (%) of blood pressure | changing rate (%) of heart rate |
|---|---|---|---|
| 60 | 0.1 | +17 | +14 |
| | 1 | +13 | +38 |
| 61 | 0.01 | +13 | +14 |
| | 0.1 | +15 | +30 |
| | 1 | +27 | +26 |
| 62 | 0.1 | - | +6 |
| | 1 | - | +13 |

Test Compound

60. 13,14-dihydro-15-keto-20-methyl-$PGE_1$ methyl ester
61. 13,14-dihydro-15-keto-16,16-difluoro-$PGE_2$ methyl ester
62. 13,14-dihydro-6,15-diketo-18-methyl-$PGE_1$ ethyl ester

Example 3

Wister male rats (9 weeks old) were used.

The rats were applied to anesthesia by intraperitoneal administration with 1.25 g/kg of urethane. The blood flow rate in carotid was determined by a stromuhr. The blood pressure and heart rate were determined according to the same manner as in Example 1.

13,14-dihydro-6,15-diketo-19-methyl-$PGE_1$ ethyl ester (sample number: 15) was dissolved in a small amount of ethanol, and then diluted with Ringer's solution more than 50 times. About 0.1 ml (1 mg drag/kg rat) of the diluted solution was administered to a rat through a polyethylene tube inserted into a femoral artery. The changes in blood flow rate (BF), blood pressure (BP), heart rate (HR) were observed, and the average of 4 experiments were shown in Fig.1.

Apparent from Fig. 1 BF, BP and HR extremely increase.

Example 4 (Recovery of blood pressure and heart rate of rats in hemorrhage shock)

Wister male rats (8 weeks old) were applied to anesthesia by subcutaneous injection with 1.5 g/kg of urethane. The blood pressure and heart rate were determined according to the same manner as in Example 1

The rats were kept for 60 minutes in a room after a blood corresponding to 1.5 % of the weight was removed.

13,14-dihydro-6,15-diketo-19-methyl-$PGE_1$ ethyl ester (compound number 15) was dissolved in Ringer's solution (0.1 ml), and the solution was intravenously administered into the above dehematized rat. Ringer's solution was administered to a control group.

The recovery rates of the blood pressure and heart rate were obtained assuming the value at 60 minutes after dehematizing being 0 % and the value before dehematizing being 100 % respectively. The average value of 5 experiments was shown in Table 3. Typically change of blood pressure with time was shown in Fig. 2, and heart rate change with time was shown in Fig. 3. In these Figures (1), (2) and (3) represent a dose of 1 mg/kg, dose of 0.5 mg/kg and control respectively.

The same procedure as the above except that the compounds of (7), (18) and (22) were used instead of the compound (15) was applied, and the effects are observed, the results of which are also shown in Table 3.

Apparent from Table 3 and Figs.2 and 3 a remarkable recovering of the blood pressure and heart rate by the administration of the test compounds was observed.

Table-3

| test compound | dose mg/kg | recovering of blood pressure % | recovering of heart rate % |
|---|---|---|---|
| 7 | 0.5 | 38 | 46 |
| | 1.0 | 63 | 62 |
| 15 | 0.5 | 39 | 62 |
| | 1.0 | 68 | 100 |
| 18 | 0.5 | 87 | 100 |
| | 1.0 | 119 | 100 |
| 22 | 0.5 | 32 | 50 |
| | 1.0 | 74 | 63 |

Example 5

Acute toxicity $LD_{50}$ of 15-keto-PGE esters to Slcddy mice was determined at oral administration (PO), subcutaneous administration (SC), intraperitoneally administration (I.P.), or intravenous administration (IV). The results were shown in Table 4.

Table 4

| test compound | administration | $LD_{50}$ (mg/kg) male | female |
|---|---|---|---|
| 22 | PO | >4000 | >4000 |
| | SC | >1000 | >1000 |
| | IP | >500 | > 500 |
| | IV | 1000 | 1000 |
| 21 | IV | >1000 | |
| 2 | IP | >1000 | |
| | IV | >1000 | |
| 15 | IV | >300 | |
| 36 | PO | >500 | |
| 37 | PO | >500 | |

Example 6 (progressing a breath)

Wister male rats (8 weeks old) were applied to anesthesia by intraperitoneal administration with 1.25 g/kg of urethane. A pick-up for breath was fixed on a top of a rat's nose, and the breath curve was recorded on a polygraph while the breath rate was counted with a tachometer.

Each test compound was dissolved in ethanol and administered to rats through a tale vein in 1 mg/kg as diluted with Ringer's solution just before use. The maximum concentration of ethanol was 2 percent. Ringers solution containing ethanol but not containing test compound was administered at every experiment as a control. The change rates (%) of the breath rate before and after administration were determined, and evaluated as progressing activity of breath. The results were shown in Table 5.

Example 7 (enteroconstriction)

Ileums were enucleated from Wister male rats, and hung up in Magunus tube. The ileums were administered with test compounds, after they were constricted several times by immersed into an acetylcholine solution of 1 x $10^{-6}$ g/ml and two constrictions were observed at the same level.

The ileum constrictions by the test compounds were evaluated by the concentration of a test compound exhibiting 50 % of the constriction achieved by the acetylcholine solution of 1 x $10^{-6}$ g/ml. This concentration was expressed by $ED_{50}$. The results were shown in Table 5.

Table 5

| test compound | progressing activity % * of breath | enteroconstriction ** |
|---|---|---|
| 1 | +34 | - |
| 2 | +47 | - |
| 4 | +48 | |
| 5 | + 4 | |
| 6 | +21 | - |
| 7 | +31 | - |
| 8 | +65 | |
| 9 | +26 | - |
| 10 | +63 | - |
| 11 | +34 | - |
| 12 | +44 | - |
| 13 | +53 | - |
| 14 | +33 | |
| 15 | +26 | - |
| 18 | +28 | - |
| 20 | +12 | - |
| 21 | +29 | - |
| 22 | +28 | - |
| 23 | +37 | - |
| 24 | +41 | - |
| 25 | +22 | - |
| 26 | +22 | - |
| 27 | +11 | - |
| 28 | +40 | - |

| test compound | progressing activity % * of breath | enteroconstriction ** |
|---|---|---|
| 29 | +11 | - |
| 30 | +61 | - |
| 31 | +40 | - |
| 32 | +41 | - |
| 33 | +40 | - |
| 34 | +31 | - |
| 35 | +37 | - |
| 38 | +47 | - |
| 40 | +16 | |
| 41 | +20 | |
| 42 | +28 | - |
| 43 | +41 | |
| 44 | +16 | - |
| 45 | +10 | - |
| 46 | +10 | - |
| 47 | +17 | - |
| 49 | +18 | - |
| 50 | +18 | - |
| 51 | +49 | - |
| 57 | +55 | + |
| 58 | +35 | |
| 59 | 0 | - |

note:

* +: exhibiting the progressing activity of breath

** -: $ED_{50} \geq 10^{-6}$

+: $ED_{50} < 10^{-6}$

slanted lines show not tested.

As apparent from the above results PGEs have progressing activity of breath in general, and 15-keto-PGEs and 13,14-dihydro-15-keto-PGEs have no enteroconstricting action as a side effect.

Example 8 (preparation of capsules)

One or more effective compound 50 mg was dissolved in ethanol (10 ml), and the solution was mixed with mannitol 18.5 g to give a wet mass. The wet mass was sifted through a 30-mesh sieve, the mass passed was dried at 30 °C for 90 minutes, and then sifted through the 30-mesh sieve again. The obtained powder was added with a microfine silica (Aerosil) 200 g and mixed. The mixed powder was filled into 100 hard gelatine capsules (No. 3) to give capsules containing 0.5 mg of the effective compound a capsule, which were soluble in the intestines.

Example 9 (preparation of a powder for injection)

Following ingredients were mixed:

| ingredients | parts by weight |
|---|---|
| effective compounds | 1 |
| Tween 80*® | 0.1 |
| mannitol | 5 |
| distilled water | 0.4 |

* : sorbitan mono oleyl ester condensed with ethyleneoxide (20 mole).

The mixture was sterilized, filtrated and then freeze-dried to give a powder for injection.

Example 10 (preparation of a liquid for injection)

Following ingredients were mixed, and sterilized to give a liquid for injection.

| ingredients: | parts by weight |
|---|---|
| effective compounds | 0.2 |
| nonionic surface active agents | 2 |
| distilled water for injection | 98 |

Example 11 (preparation of a powder for oral administration)

Following ingredients were mixed to give a powder for oral administration.

| ingredients: | parts by weight |
|---|---|
| effective compounds | 5 |
| light dry silicic acid | 5 |
| Abicel® | 20 |
| lactose | 70 |

Example 12 ( preparation of a soft capsule)

Following ingredients were mixed and filled up into a soft capsule.

| ingredients | parts by weight |
|---|---|
| effective compounds | 1 |
| Pasenat | 999 |

Synthetic Chart I

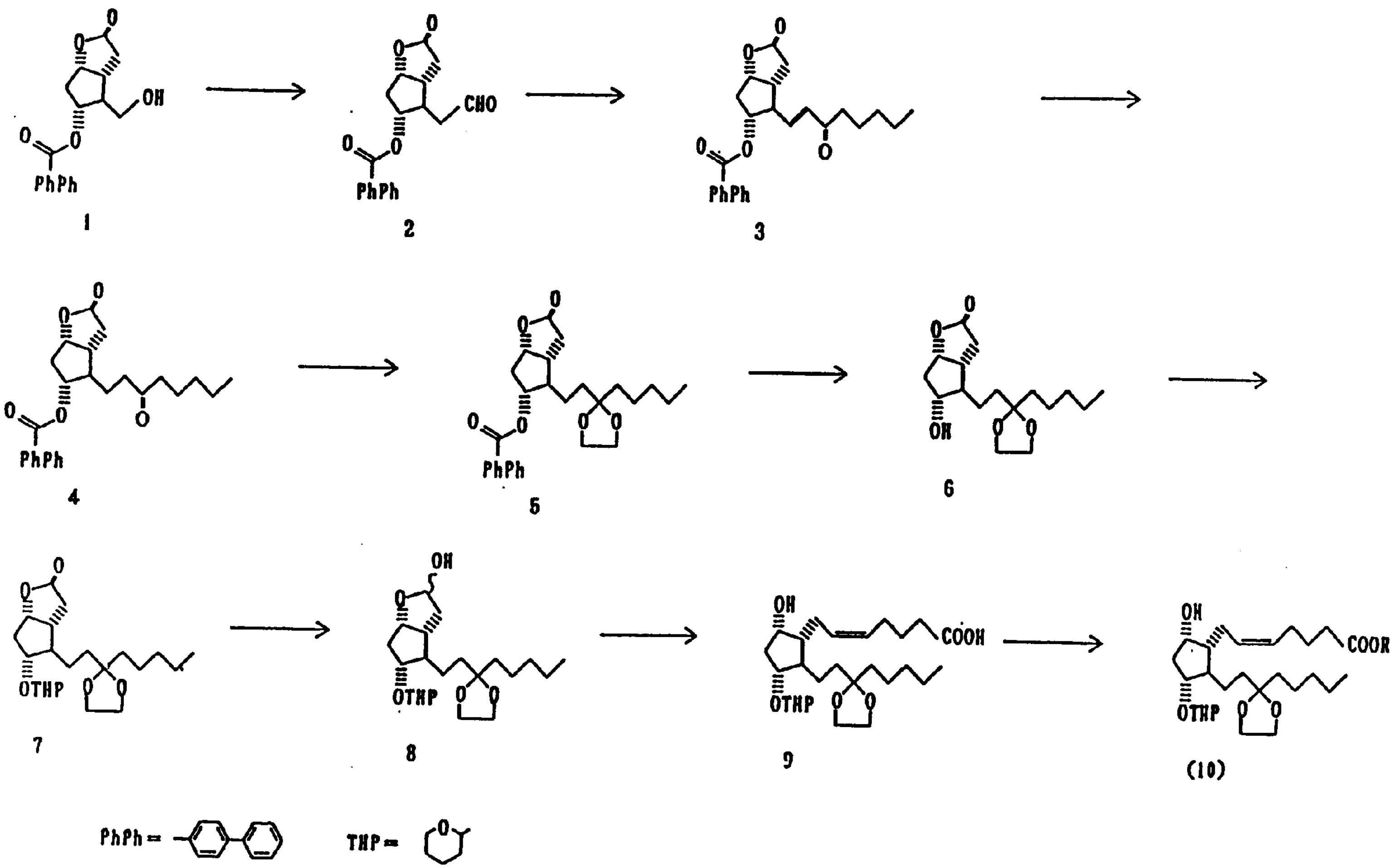

1 2 3 4 5 6 7 8 9 (10)

PhPh = THP =

Synthetic Chart I (Continued)

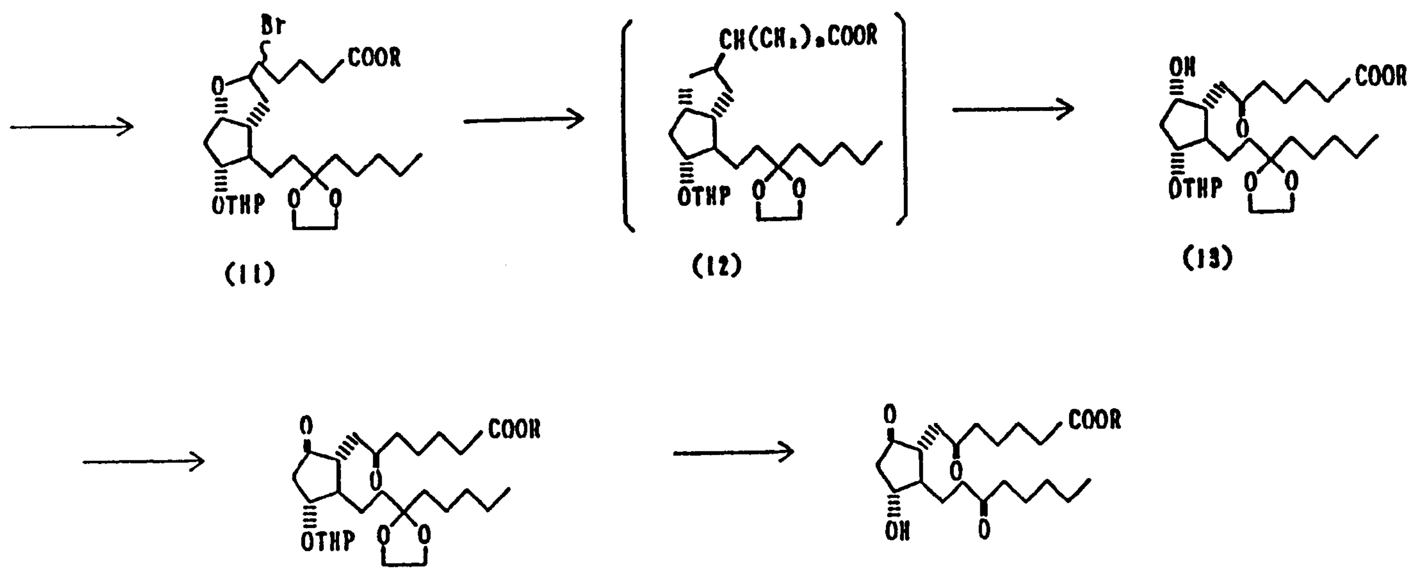

(11) (12) (13)

(14) (15)

Synthetic Chart II

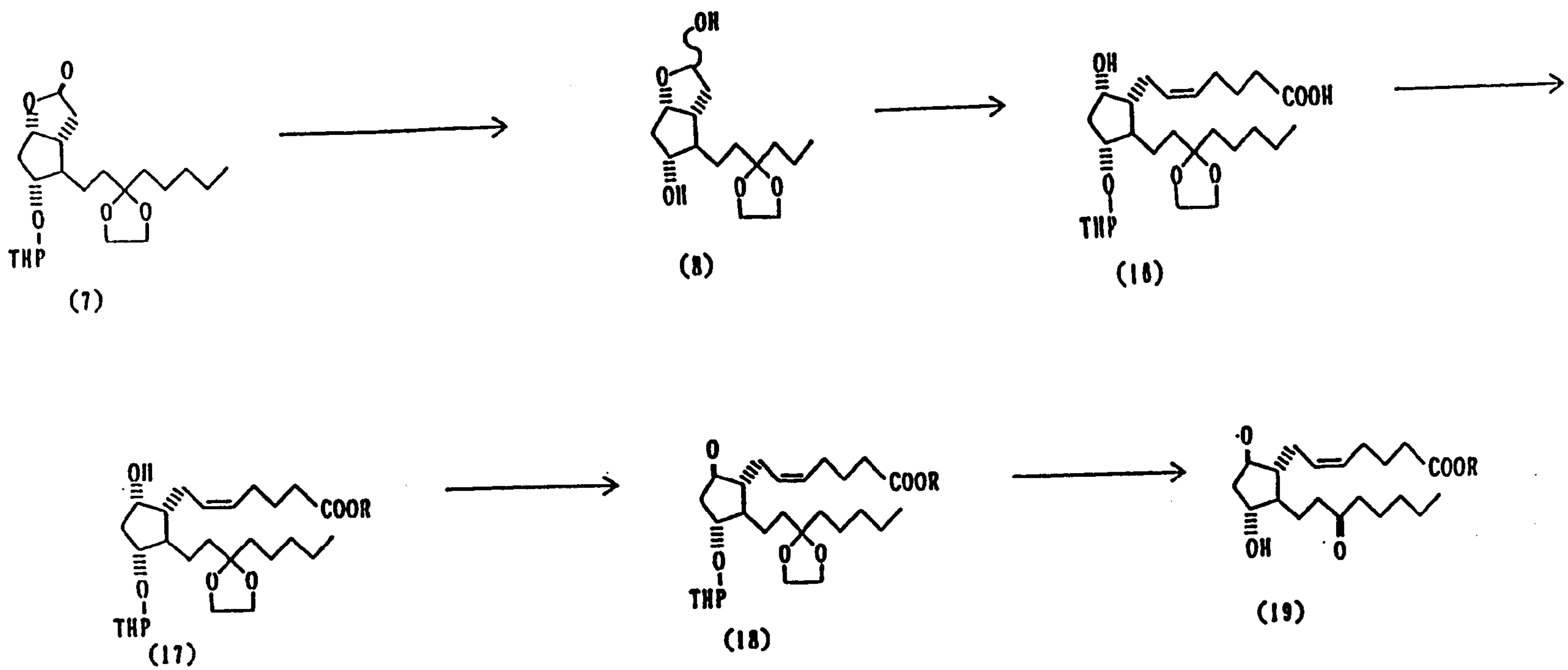

(7)

(8)

(16)

(17)

(18)

(19)

Synthetic Chart III

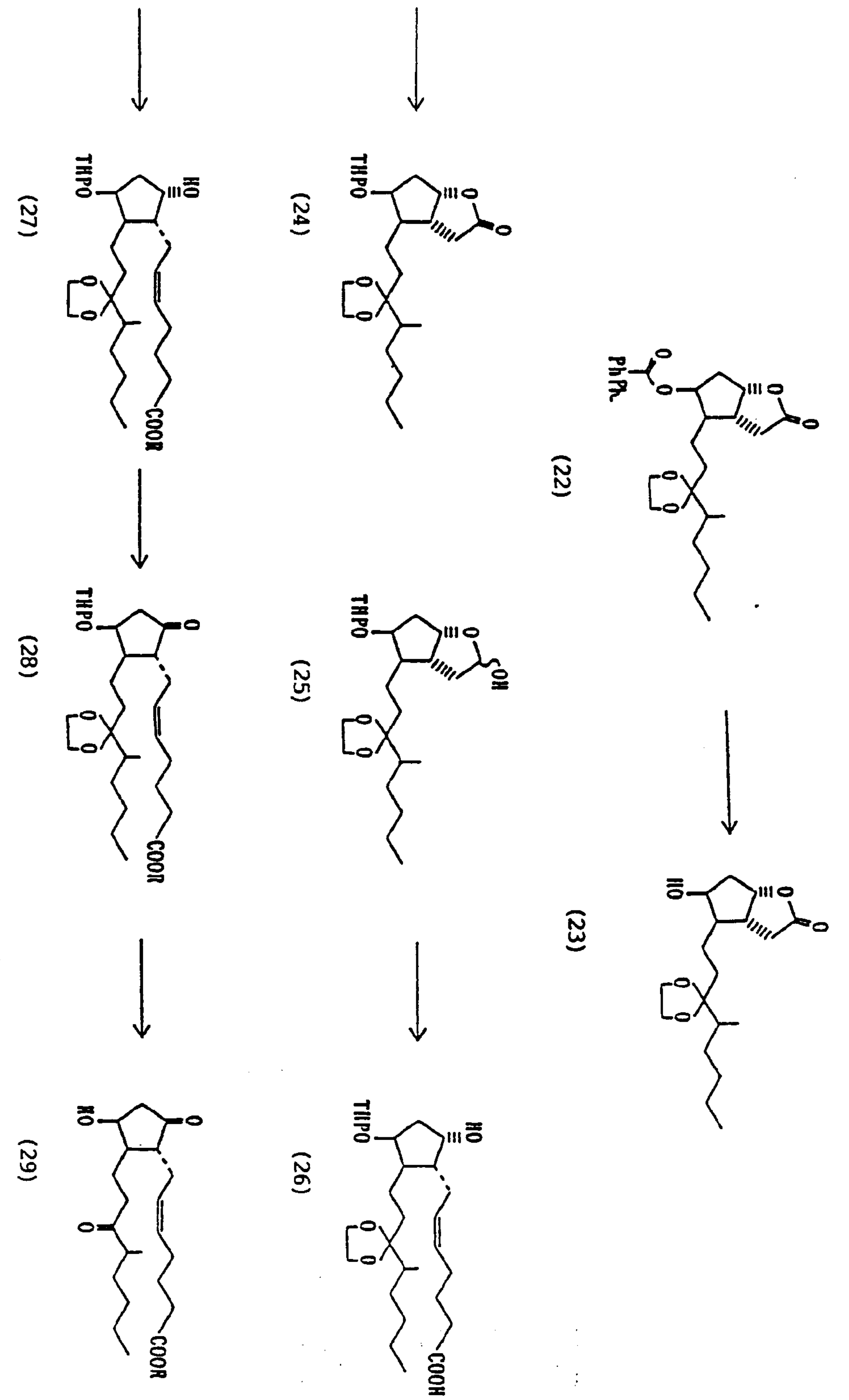

(22) (23) (24) (25) (26) (27) (28) (29)

## Claims

**Claims for the following Contracting States : AT, BE, CH, DE, FR, GB, IT, LI, LU, NL, SE:**

1. The use of a composition comprising a 15-keto-PGE ester and a pharmaceutically acceptable carrier for the manufacture of a medicament effective for increasing blood pressure.

2. The use of a composition comprising a 15-keto-PGE ester and a pharmaceutically acceptable carrier for the manufacture of a medicament effective for increasing heart rate.

3. The use of a composition comprising a 15-keto-PGE ester and a pharmaceutically acceptable carrier for the manufacture of a medicament effective for increasing blood flow rate.

4. The use of a composition comprising a 15-keto-PGE ester and a pharmaceutically acceptable carrier for the manufacture of a medicament effective for progressing breath.

5. The use according to any one of Claims 1 to 4, in which the 15-keto-PGE ester is represented by the following formula:

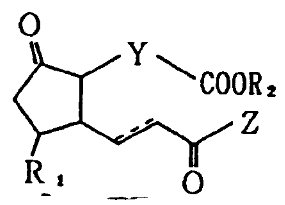

wherein $R_1$ is a hydroxyl group, a hydroxyalkyl group, or an alkyl group; Y is a saturated or an unsaturated hydrocarbon moiety having 2 - 6 carbon atoms wherein a portion of carbon atoms constituting the hydrocarbon moiety may be carbonyl or the hydrocarbon moiety may be substituted with other atoms or groups; Z is a saturated or an unsaturated hydrocarbon moiety having 1 - 10 carbon atoms which may constitute a ring or the hydrocarbon moiety may be substituted with other atoms or groups; $COOR_2$ is a physiologically acceptable ester group thereof.

6. The use according to Claim 5, in which the hydrocarbon moiety represented by Z is substituted with one or more group(s) or atom(s) selected from a hydroxyl group, an alkyl group, a phenyl group, an alkoxy group and a phenoxy group or a halogen atom.

7. The use according to Claim 5, in which Z represents a formula:

$$-CH_2CH_2CH_2CH(R_1)(R_2):$$

wherein $R_1$ is a hydrogen atom or a methyl group, and $R_2$ is a methyl group, an ethyl group or a propyl group.

8. The use according to Claim 5, wherein Z is a fluoroalkyl group.

9. The use according to any one of Claims 1 to 4, wherein the 15-keto-PGE ester is a 13,14-dihydro-15-keto-PGE ester.

10. The use according to any one of Claims 1 to 4, wherein the 15-keto-PGE is a 6,15-diketo-PGE ester.

11. The use according to any one of Claims 1 to 4, wherein the 15-keto-PGE is a 15-keto-3-alkyl-PGE ester.

12. The use according to any one of Claims 1 to 4, wherein the 15-keto-PGE is a 15-keto-16-alkyl-PGE ester.

13. The use according to any one of Claims 1 to 4, wherein the 15-keto-PGE is a 15-keto-16-halogen-PGE ester.

14. The use according to any one of Claims 1 to 4, wherein the 15-keto-PGE is a 15-keto-19-alkyl-PGE ester.

15. The use according to any one of Claims 1 to 4, wherein the 15-keto-PGE is a 15-keto-20-alkyl-PGE ester.

**Claims for the following Contracting States : ES, GR**

1. A method for the manufacture of a medicament effective for increasing blood pressure comprising admixing a 15-keto-PGE ester and a pharmaceutically acceptable carrier.

2. A method for the manufacture of a medicament effective for increasing heart rate comprising admixing a 15-keto-PGE ester and a pharmaceutically acceptable carrier.

3. A method for the manufacture of a medicament effective for increasing blood flow rate comprising admixing a 15-keto-PGE ester and a pharmaceutically acceptable carrier.

4. A method for the manufacture of a medicament effective for progressing breath comprising admixing a 15-keto-PGE ester and a pharmaceutically acceptable carrier.

5. The method according to any one of Claims 1 to 4, in which the 15-keto-PGE ester is represented by the following formula:

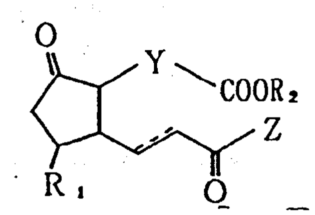

wherein $R_1$ is a hydroxyl group, a hydroxyalkyl group, or an alkyl group; Y is a saturated or an unsaturated hydrocarbon moiety having 2 - 6 carbon atoms wherein a portion of carbon atoms constituting the hydrocarbon moiety may be carbonyl or the hydrocarbon moiety may be substituted with other atoms or groups; Z is a saturated or an unsaturated hydrocarbon moiety having 1 - 10 carbon atoms which may constitute a ring or the hydrocarbon moiety may be substituted with other atoms or groups; $COOR_2$ is a physiologically acceptable ester group thereof.

6. The method according to Claim 5, in which the hydrocarbon moiety represented by Z is substituted with one or more group(s) or atom(s) selected from a hydroxyl group, an alkyl group, a phenyl group, an alkoxy group and a phenoxy group or a halogen atom.

7. The method according to Claim 5, in which Z represents a formula:

$$-CH_2CH_2CH_2CH(R_1)(R_2):$$

wherein $R_1$ is a hydrogen atom or a methyl group, and $R_2$ is a methyl group, an ethyl group or a propyl group.

8. The method according to Claim 5, wherein Z is a fluoroalkyl group.

9. The method according to any one of Claims 1 to 4, wherein the 15-keto-PGE ester is a 13,14-dihydro-15-keto-PGE ester.

10. The method according to any one of Claims 1 to 4, wherein the 15-keto-PGE is a 6,15-diketo-PGE ester.

11. The method according to any one of Claims 1 to 4, wherein the 15-keto-PGE is a 15-keto-3-alkyl-PGE ester.

12. The method according to any one of Claims 1 to 4, wherein the 15-keto-PGE is a 15-keto-16-alkyl-PGE ester.

13. The method according to any one of Claims 1 to 4, wherein the 15-keto-PGE is a 15-keto-16-halogen-PGE ester.

14. The method according to any one of Claims 1 to 4, wherein the 15-keto-PGE is a 15-keto-19-alkyl-PGE ester.

15. The method according to any one of Claims 1 to 4, wherein the 15-keto-PGE is a 15-keto-20-alkyl-PGE ester.

## Patentansprüche

### Patentansprüche für folgende Vertragsstaaten : AT, BE, CH, DE, FR, GB, IT, LI, LU, NL, SE

1. Verwendung eines Präparats, das einen 15-Keto-PGE-ester und einen pharmazeutisch annehmbaren Träger enthält, für die Herstellung eines Arzneimittels, das für die Erhöhung des Blutdruckes wirksam ist.

2. Verwendung eines Präparats, das einen 15-Keto-PGE-ester und einen pharmazeutisch annehmbaren Träger enthält, für die Herstellung eines Arzneimittels, das für die Erhöhung des Herzschlags wirksam ist.

3. Verwendung eines Präparats, das einen 15-Keto-PGE-ester und einen pharmazeutisch annehmbaren Träger enthält, für die Herstellung eines Arzneimittels, das für die Erhöhung der Durchblutungsgeschwindigkeit wirksam ist.

4. Verwendung eines Präparats, das einen 15-Keto-PGE-ester und einen pharmazeutisch annehmbaren Träger enthält, für die Herstellung eines Arzneimittels, das für das Fortschreiten des Atmens bzw. das Weiteratmen wirksam ist.

5. Verwendung nach irgendeinem der Ansprüche 1 bis 4, dadurch **gekennzeichnet**, daß der 15-Keto-PGE-ester durch die folgende Formel

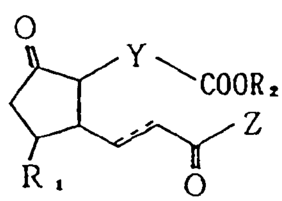

dargestellt wird, worin
$R_1$ eine Hydroxylgruppe, eine Hydroxyalkylgruppe oder eine Alkylgruppe bedeutet;
Y eine gesättigte oder ungesättigte Kohlenwasserstoffgruppierung mit 2 bis 6 Kohlenstoffatomen bedeutet, wobei ein Teil der Kohlenstoffatome, die die Kohlenwasserstoffgruppierung darstellt, Carbonyl sein kann, oder wobei die Kohlenwasserstoffgruppierung mit anderen Atomen oder Gruppen substituiert sein kann;
Z eine gesättigte oder ungesättigte Kohlenwasserstoffgruppierung mit 1 bis 10 Kohlenstoffatomen bedeutet, die ein Ring sein kann oder wobei die Kohlenwasserstoffgruppierung mit anderen Atomen oder Gruppen substituiert sein kann;
$COOR_2$ eine physiologisch annehmbare Estergruppe davon bedeutet.

6. Verwendung nach Anspruch 5, dadurch **gekennzeichnet**, daß die durch Z dargestellte Kohlenwasserstoffgruppierung durch eine oder mehrere Gruppe(n) oder Atom(e), ausgewählt aus einer Hydroxylgruppe, einer Alkylgruppe, einer Phenylgruppe, einer Alkoxygruppe und einer Phenoxygruppe oder durch ein Halogenatom substituiert ist.

7. Verwendung nach Anspruch 5, dadurch **gekennzeichnet**, daß Z die Formel

$$-CH_2CH_2CH_2CH(R_1)(R_2)$$

besitzt, worin $R_1$ ein Wasserstoffatom oder eine Methylgruppe bedeutet und $R_2$ eine Methylgruppe, eine Ethylgruppe oder eine Propylgruppe bedeutet.

8. Verwendung nach Anspruch 5, dadurch **gekennzeichnet**, daß Z eine Fluoralkylgruppe bedeutet.

9. Verwendung nach einem der Ansprüche 1 bis 4, dadurch **gekennzeichnet**, daß 15-Keto-PGE-ester ein 13,14-Dihydro-15-keto-PGE-ester ist.

10. Verwendung nach einem der Ansprüche 1 bis 4, dadurch **gekennzeichnet**, daß 15-Keto-PGE ein 6,15-Di-keto-PGE-ester ist.

11. Verwendung nach einem der Ansprüche 1 bis 4, dadurch **gekennzeichnet,** daß 15-Keto-PGE ein 15-Keto-3-alkyl-PGE-ester ist.

12. Verwendung nach einem der Ansprüche 1 bis 4, dadurch **gekennzeichnet**, daß 15-Keto-PGE ein 15-Keto-16-alkyl-PGE-ester ist.

13. Verwendung nach einem der Ansprüche 1 bis 4, dadurch **gekennzeichnet**, daß 15-Keto-PGE ein 15-Keto-16-halogen-PGE-ester ist.

14. Verwendung nach einem der Ansprüche 1 bis 4, dadurch **gekennzeichnet**, daß 15-Keto-PGE ein 15-Keto-19-alkyl-PGE-ester ist. 19-alkyl-PGE-ester ist.

15. Verwendung nach einem der Ansprüche 1 bis 4, dadurch **gekennzeichnet**, daß 15-Keto-PGE ein 15-Keto-20-alkyl-PGE-ester ist.

**Patentansprüche für folgende Vertragsstaaten : ES, GR**

1. Verfahren zur Herstellung eines Arzneimittels, das bei der Erhöhung des Blutdrucks wirksam ist, dadurch **gekennzeichnet**, daß ein 15-Keto-PGE-ester und ein pharmazeutisch annehmbarer Träger vermischt werden.

2. Verfahren zur Herstellung eines Arzneimittels, das für die Erhöhung des Herzschlags wirksam ist, dadurch **gekennzeichnet**, daß ein 15-Keto-PGE-ester und ein pharmazeutisch annehmbarer Träger vermischt werden.

3. Verfahren zur Herstellung eines Arzneimittels, das für die Erhöhung der Durchblutungsgeschwindigkeit wirksam ist, dadurch **gekennzeichnet**, daß ein 15-Keto-PGE-ester und ein pharmazeutisch annehmbarer Träger vermischt werden.

4. Verfahren zur Herstellung eines Arzneimittels, das für das Fortschreiten des Atmens bzw. das Weiteratmen wirksam ist, dadurch **gekennzeichnet**, daß ein 15-Keto-PGE-ester und ein pharmazeutisch annehmbarer Träger vermischt werden.

5. Verfahren nach einem der Ansprüche 1 bis 4, dadurch **gekennzeichnet,** daß der 15-Keto-PGE-ester durch die folgende Formel

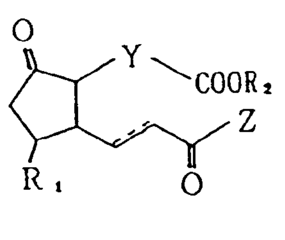

dargestellt wird, worin
$R_1$ eine Hydroxylgruppe, eine Hydroxyalkylgruppe oder eine Alkylgruppe bedeutet;
Y eine gesättigte oder ungesättigte Kohlenwasserstoffgruppierung mit 2 bis 6 Kohlenstoffatomen bedeutet, wobei ein Teil der Kohlenstoffatome, die die Kohlenwasserstoffgruppierung darstellt, Carbonyl sein kann, oder wobei die Kohlenwasserstoffgruppierung mit anderen Atomen oder Gruppen substituiert sein kann;
Z eine gesättigte oder ungesättigte Kohlenwasserstoffgruppierung mit 1 bis 10 Kohlenstoffatomen bedeutet, die ein Ring sein kann oder wobei die Kohlenwasserstoffgruppierung mit anderen Atomen oder Gruppen substituiert sein kann;

$COOR_2$ eine physiologisch annehmbare Estergruppe davon bedeutet.

6. Verfahren nach Anspruch 5, dadurch **gekennzeichnet**, daß die durch Z dargestellte Kohlenwasserstoffgruppierung durch eine oder mehrere Gruppe(n) oder Atom(e), ausgewählt aus einer Hydroxylgruppe, einer Alkylgruppe, einer Phenylgruppe, einer Alkoxygruppe und einer Phenoxygruppe, oder durch ein Halogenatom substituiert ist.

7. Verfahren nach Anspruch 5, dadurch **gekennzeichnet**, daß Z die Formel

$$-CH_2CH_2CH_2CH(R_1)(R_2)$$

besitzt, worin $R_1$ ein Wasserstoffatom oder eine Methylgruppe bedeutet, und $R_2$ eine Methylgruppe, eine Ethylgruppe oder eine Propylgruppe bedeutet.

8. Verfahren nach Anspruch 5, dadurch **gekennzeichnet**, daß Z eine Fluoralkylgruppe bedeutet.

9. Verfahren nach einem der Ansprüche 1 bis 4, dadurch **gekennzeichnet**, daß der 15-Keto-PGE-ester ein 13,14-Dihydro-15-keto-PGE-ester ist.

10. Verfahren nach einem der Ansprüche 1 bis 4, dadurch **gekennzeichnet**, daß 15-Keto-PGE ein 6,15-Diketo-PGE-ester ist.

11. Verfahren nach einem der Ansprüche 1 bis 4, dadurch **gekennzeichnet**, daß 15-Keto-PGE ein 15-Keto-3-alkyl-PGE-ester ist.

12. Verfahren nach einem der Ansprüche 1 bis 4, dadurch **gekennzeichnet**, daß 15-Keto-PGE ein 15-Keto-16-alkyl-PGE-ester ist.

13. Verfahren nach einem der Ansprüche 1 bis 4, dadurch **gekennzeichnet**, daß 15-Keto-PGE ein 15-Keto-16-halogen-PGE-ester ist.

14. Verfahren nach einem der Ansprüche 1 bis 4, dadurch **gekennzeichnet**, daß 15-Keto-PGE ein 15-Keto-19-alkyl-PGE-ester ist.

15. Verfahren nach einem der Ansprüche 1 bis 4, dadurch **gekennzeichnet**, daß 15-Keto-PGE ein 15-Keto-20-alkyl-PGE-ester ist.

## Revendications

### Revendications pour les Etats contractants suivants : AT, BE, CH, DE, FR, GB, IT, LI, LU, NL, SE

1. Utilisation d'une composition comprenant un ester de 15-céto-PGE et un vecteur acceptable sur le plan pharmaceutique pour la fabrication d'un médicament efficace pour augmenter la pression sanguine.

2. Utilisation d'une composition comprenant un ester de 15-céto-PGE et un vecteur acceptable sur le plan pharmaceutique pour la fabrication d'un médicament efficace pour augmenter le rythme cardiaque.

3. Utilisation d'une composition comprenant un ester de 15-céto-PGE et un vecteur acceptable sur le plan pharmaceutique pour la fabrication d'un médicament efficace pour augmenter le débit sanguin.

4. Utilisation d'une composition comprenant un ester de 15-céto-PGE et un vecteur acceptable sur le plan pharmaceutique pour la fabrication d'un médicament efficace pour faire progresser la respiration.

5. Utilisation selon l'une quelconque des revendications 1 à 4, dans laquelle l'ester de 15-céto-PGE est représenté par la formule suivante :

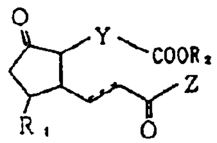

dans laquelle $R_1$ est un groupe hydroxyle, un groupe hydroxyalkyle ou un groupe alkyle; Y est un groupe hydrocarbure saturé ou insaturé ayant 2 - 6 atomes de carbone, où une portion des atomes de carbone constituant le groupe hydrocarbure peut être un carbonyle, ou le groupe hydrocarbure peut être substitué avec d'autres atomes ou groupes; Z est un groupe hydrocarbure saturé ou insaturé ayant 1 - 10 atomes de carbone qui peuvent constituer un cycle ou le groupe hydrocarbure peut être substitué par d'autres atomes ou groupes; $COOR_2$ est un groupe ester acceptable du point de vue physiologique.

6. Utilisation selon la revendication 5, dans laquelle le groupe hydrocarbure représenté par Z est substitué avec un ou plusieurs groupes ou atomes choisis parmi les groupes hydroxyle, alkyle, phényle, alcoxy, phénoxy ou halogène.

7. Utilisation selon la revendication 5, dans laquelle Z représente une formule :

$$-CH_2CH_2CH_2CH(R_1)(R_2) :$$

dans laquelle $R_1$ est un atome d'hydrogène ou un groupe méthyle et $R_2$ est un groupe méthyle, un groupe éthyle ou un groupe propyle.

8. Utilisation selon la revendication 5, dans laquelle Z est un groupe fluoroalkyle.

9. Utilisation selon l'une quelconque des revendications 1 à 4, dans laquelle l'ester de 15-céto-PGE est un ester de 13,14-dihydro-15-céto-PGE.

10. Utilisation selon l'une quelconque des revendications 1 à 4, dans laquelle la 15-céto-PGE est un ester de 6,15-di-céto-PGE.

11. Utilisation selon l'une quelconque des revendications 1 à 4, dans laquelle la 15-céto-PGE est un ester de 15-céto-3-alkyl-PGE.

12. Utilisation selon l'une quelconque des revendications 1 à 4, dans laquelle la 15-céto-PGE est un ester de 15-céto-16-alkyl-PGE.

13. Utilisation selon l'une quelconque des revendications 1 à 4, dans laquelle la 15-céto-PGE est un ester de 15-céto-16-halogène-PGE.

14. Utilisation selon l'une quelconque des revendications 1 à 4, dans laquelle la 15-céto-PGE est un ester de 15-céto-19-alkyl-PGE.

15. Utilisation selon l'une quelconque des revendications 1 à 4, dans laquelle la 15-céto-PGE est un ester de 15-céto-20-alkyl-PGE.

**Revendications pour les Etats contractants suivants : ES, GR**

1. Méthode pour la fabrication d'un médicament efficace pour augmenter la pression sanguine comprenant de mélanger un ester de 15-céto-PGE et un vecteur acceptable sur le plan pharmaceutique.

2. Méthode pour la fabrication d'un médicament efficace pour augmenter le rythme cardiaque comprenant de mélanger un ester de 15-céto-PGE et un vecteur acceptable sur le plan pharmaceutique.

3. Méthode pour la fabrication d'un médicament efficace pour augmenter le débit sanguin comprenant de mélanger un ester de 15-céto-PGE et un vecteur acceptable sur le plan pharmaceutique.

4. Méthode pour la fabrication d'un médicament efficace pour faire progresser la respiration comprenant de mélanger un ester de 15-céto-PGE et un vecteur acceptable sur le plan pharmaceutique.

5. Méthode selon l'une quelconque des revendications 1 à 4, dans laquelle l'ester de 15-céto-PGE est représenté par la formule suivante :

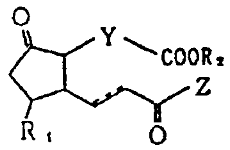

dans laquelle $R_1$ est un groupe hydroxyle, un groupe hydroxyalkyle ou un groupe alkyle; Y est un groupe hydrocarbure saturé ou insaturé ayant 2 - 6 atomes de carbone, où une portion des atomes de carbone constituant le groupe hydrocarbure peut être un carbonyle, ou le groupe hydrocarbure peut être substitué avec d'autres atomes ou groupes; Z est un groupe hydrocarbure saturé ou insaturé ayant 1 - 10 atomes de carbone qui peuvent constituer un cycle ou le groupe hydrocarbure peut être substitué avec d'autres atomes ou groupes; $COOR_2$ est un groupe ester acceptable du point de vue physiologique.

**6.** Méthode selon la revendication 5, dans laquelle le groupe hydrocarbure représenté par Z est substitué avec un ou plusieurs groupes ou atomes choisis parmi les groupes hydroxyle, alkyle, phényle, alcoxy, phénoxy ou halogène.

**7.** Méthode selon la revendication 5, dans laquelle Z représente une formule :

$$-CH_2CH_2CH_2CH(R_1)(R_2) :$$

dans laquelle $R_1$ est un atome d'hydrogène ou un groupe méthyle et $R_2$ est un groupe méthyle, un groupe éthyle ou un groupe propyle.

**8.** Méthode selon la revendication 5, dans laquelle Z est un groupe fluoroalkyle.

**9.** Méthode selon l'une quelconque des revendications 1 à 4, dans laquelle l'ester de 15-céto-PGE est un ester de 13,14-dihydro-15-céto-PGE.

**10.** Méthode selon l'une quelconque des revendications 1 à 4, dans laquelle la 15-céto-PGE est un ester de 6,15-di-céto-PGE.

**11.** Méthode selon l'une quelconque des revendications 1 à 4, dans laquelle la 15-céto-PGE est un ester de 15-céto-3-alkyl-PGE.

**12.** Méthode selon l'une quelconque des revendications 1 à 4, dans laquelle la 15-céto-PGE est un ester de 15-céto-16-alkyl-PGE.

**13.** Méthode selon l'une quelconque des revendications 1 à 4, dans laquelle la 15-céto-PGE est un ester de 15-céto-16-halogène-PGE.

**14.** Méthode selon l'une quelconque des revendications 1 à 4, dans laquelle la 15-céto-PGE est un ester de 15-céto-19-alkyl-PGE.

**15.** Méthode selon l'une quelconque des revendications 1 à 4, dans laquelle la 15-céto-PGE est un ester de 15-céto-20-alkyl-PGE.

Fig. 1

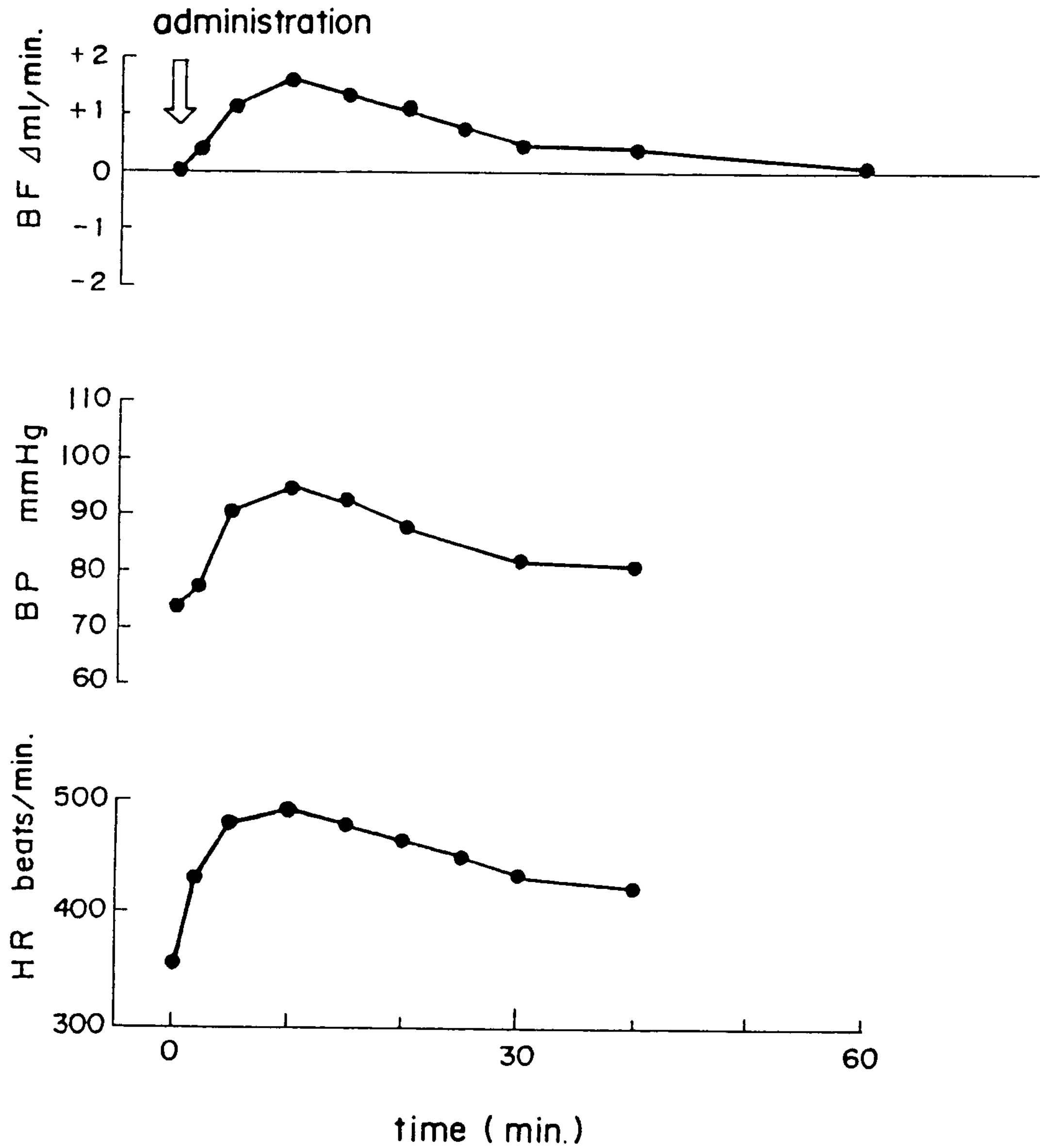
administration
BF ⊿ml/min.
+2
+1
0
-1
-2
BP mmHg
110
100
90
80
70
60
HR beats/min.
500
400
300
0
30
60
time (min.)

*Fig. 2*

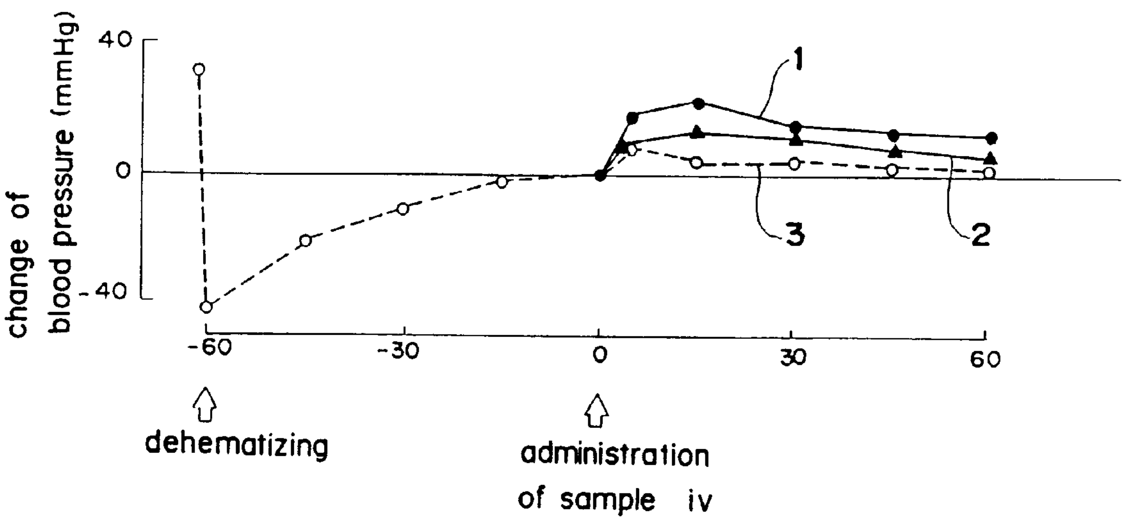
change of
blood pressure (mmHg)
40
0
-40
-60
-30
0
30
60
1
2
3
dehematizing
administration
of sample iv

*Fig. 3*

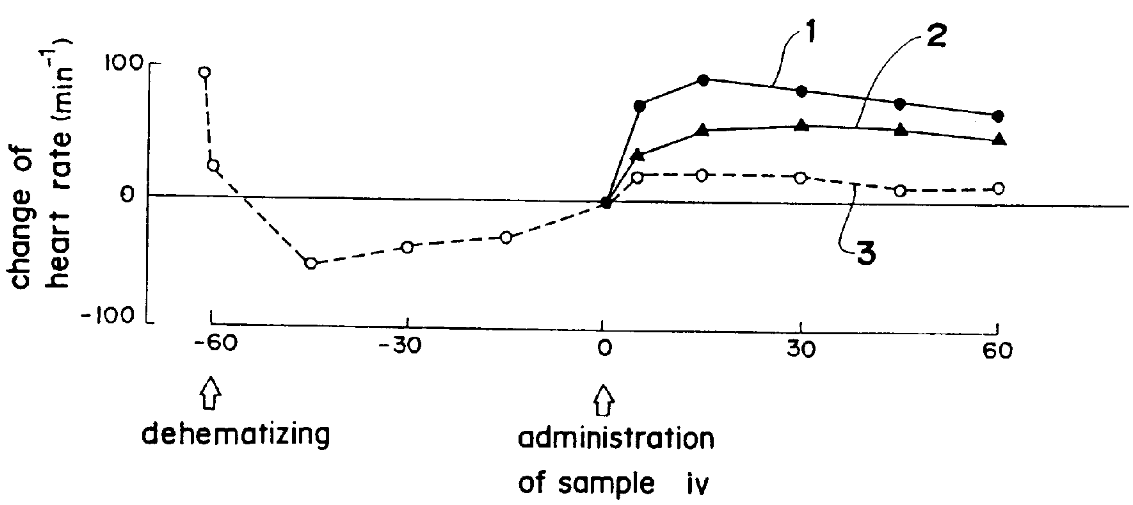
change of
heart rate (min$^{-1}$)
100
0
-100
-60
-30
0
30
60
1
2
3
dehematizing
administration
of sample iv